# EUROPÄISCHE PATENTANMELDUNG

(11) **EP 3 251 639 A1**
(43) Veröffentlichungstag der Anmeldung: **06.12.2017**
(21) Anmeldenummer: 16173025.4
(22) Anmeldetag: 05.06.2016
(51) Int. Cl.: A61F 9/013

(54) **VORRICHTUNG ZUR INDENTATION UND MARKIERUNG DER SKLERA**

(71) Anmelder: Justus-Liebig-Universität Gießen, 35390 Gießen (DE)
(72) Erfinder: LYTVYNCHUK, Lyubomyr, 35392 Gießen (DE)
(74) Vertreter: Stumpf, Peter

(57) **Zusammenfassung**

Die vorliegende Erfindung betrifft eine Vorrichtung (1) zur Indentation und Markierung der Sklera für die Behandlung von Erkrankungen der Retina eines Auges, umfassend- einen Handgriff (2), geeignet zum Halten der Vorrichtung mit einer Hand,- einen Fortsatz (2) mit einer Spitze (4), geeignet für die Durchführung einer Indentation,- eine Markierungseinheit (10) mit einem Auslösemechanismus (7), geeignet für die Durchführung einer Markierungwobei- der Fortsatz mit der Spitze an einem Ende des Handgriffs angebracht ist,- die Spitze ein Reservoir (8) für einen Farbstoff aufweist,- die Markierungseinheit an der Spitze endet,- die Markierungseinheit sich in einer Position 1 befindet, in der Farbstoff aus dem Reservoir an der Spitze der Vorrichtung nicht austreten kann,- der Auslösemechanismus an einer geeigneten Stelle am Handgriff angebracht ist, wobei bei der Aktivierung des Auslösemechanismus die Markierungseinheit in eine Position 2 bewegt wird, wodurch der Farbstoff aus dem Reservoir an der Spitze der Vorrichtung austritt.

## Beschreibung

Die vorliegende Erfindung betrifft eine Vorrichtung (augenchirurgisches Instrument) zur Indentation und Markierung der Sklera für die Behandlung von Erkrankungen des Auges, insbesondere Erkrankungen der Retina und der Chorioidea. Die Sklera ist die Lederhaut bei Augen von Wirbeltieren. Die Retina ist die Netzhaut bei Augen von Wirbeltieren. Die Chorioidea ist die Aderhaut bei Augen von Wirbeltieren. Es handelt sich bei dieser Vorrichtung also um ein Indentations- und Markierungsinstrument. Die erfindungsgemäße Vorrichtung wird bei chirurgischen Eingriffen an Augen eingesetzt, insbesondere bei Eingriffen an der Retina, der Chorioidea und bei plastischen chirurgischen Eingriffen.

Die erfindungsgemäße Vorrichtung ist für die Indentation und Markierung der Sklera von Augen bei Wirbeltieren geeignet. Zu den Wirbeltieren gehören Amphibien, Reptilien, Fische, Vögel und Säugetiere, und auch der Mensch.

Bei vielen Erkrankungen der Retina und der Chorioidea ist es wichtig, die genaue Lokalisation der erkrankten Stellen auf der Sklera zu markieren, damit bei einem operativen Eingriff an den richtigen Stellen über einen Zugang durch die Sklera die Retina oder Chorioidea therapiert wird. Dieses Vorgehen wird deshalb eingesetzt, weil bei operativen Eingriffen an der Retina oder Chorioidea erst der Augapfel aus der Augenhöhle herausgelagert (rotiert) wird und dann von außen durch die Sklera und die Chorioidea der operative Zugang zur Retina geschaffen wird. Bei diesem Vorgehen kann die Retina selber mittels indirekter Ophthalmoskopie betrachtet werden.

Als Indentation der Sklera bezeichnet man das leichte Eindrücken der Sklera von außen in Richtung zur Retina hin bei einem aus der Augenhöhle herausgelagertem Augapfel. Für dieses Eindrücken werden spezielle augenchirurgische Instrumente verwendet, welche Indentatoren, Lokalisatoren, Deller oder Depressoren genannt werden. Durch dieses Drücken von außen mit einem Indentator, Lokalisator, Deller oder Depressor auf die Sklera wird die Sklera auf die darunterliegende Chorioidea und Retina gedrückt, so dass an der betreffenden Stelle eine Ausbuchtung an der Retina zu erkennen ist. Diese Ausbuchtung kann mittels indirekter Ophthalmoskopie erkannt und lokalisiert werden. Das Drücken auf die Sklera von außen wird auch "scleral indentation" oder "scleral depression" genannt.

Mit Hilfe der Indentation der Sklera können bestimmte Abschnitte der Retina besser in Augenschein genommen und mittels indirekter Ophthalmoskopie untersucht werden als ohne Indentation. Dies betrifft vor allem die peripheren Abschnitte der Retina. Die Indentation in Kombination mit der indirekten Ophthalmoskopie wird beispielsweise zur Diagnose von Löchern, Rissen, Ablösungen oder Tumoren an der Retina angewendet. Für die Indentation wurden verschiedene augenchirurgische Instrumente entwickelt.

Nach der Diagnose der krankhaft veränderten Stellen an der Retina sollen genau diese veränderten Stellen so markiert werden, dass sie an der Sklera erkennbar sind, weil von der Sklera aus der operative Zugang zur Retina erfolgt. Bei Erkrankungen der Retina besteht nämlich die Therapie häufig darin, durch die Sklera hindurch einen operativen Eingriff vorzunehmen, beispielsweise eine Kältebehandlung zur Therapie einer Retinaablösung oder die chirurgische Entfernung eines Retinatumors. Dazu muss aber an der richtigen Stelle der Sklera der Zugang zur Retina erfolgen.

Beispielsweise kann ein Riss in der Retina durch eine Kälteapplikation (Kryotherapie) auf der entsprechenden Stelle der darüber liegenden Sklera behandelt werden. Aus dem Grund ist es oft notwendig, an der Sklera jene Stellen zu markieren, unter denen sich die Veränderungen an der Retina befinden. Zur Identifizierung und Markierung der entsprechenden Stellen an der Sklera wird üblicherweise folgendes Verfahren eingesetzt: Nach dem Herauslagern (Rotieren) des Augapfels aus der Augenhöhle wird eine indirekte Ophthalmoskopie zur Betrachtung der Retina durchgeführt. Gleichzeitig wird eine Indentation der Sklera vorgenommen. An den Stellen, wo die Sklera durch die Indentation eingedrückt wird, ist bei der indirekten Ophthalmoskopie eine Ausbuchtung der Retina zu erkennen. Wenn diese Ausbuchtung genau an der Stelle der Retinaveränderung (beispielsweise Loch, Riss, Ablösung oder Tumor) zu sehen ist, dann bedeutet das, dass genau an dieser Stelle auf der Sklera indentatiert wird. Genau diese Stelle wird dann auf der Sklera mit einem Farbstoff markiert, damit dann der operierende Arzt weiß, dass er genau an dieser Stelle den operativen Zugang zur Retina durch die Sklera hindurch vornehmen muss.

### Stand der Technik

Für die Indentation der Sklera gibt es eine Vielzahl an Instrumenten (auch Lokalisatoren, Depressoren, Deller oder Indentoren genannt), die im Wesentlichen folgende Bauteile aufweisen: Einen Handgriff und einen Fortsatz mit einem Ende, welches für das Drücken auf die Sklera ausgebildet ist. Die Enden können rund, tropfenförmig, kugelförmig oder schleifenförmig sein oder eine andere Form aufweisen. Es gibt auch Indentoren, die an Stelle eines Handgriffes einen Fingerhut aufweisen und somit auf eine Fingerkuppe gesteckt werden können. Indentoren sind im Allgemeinen aus Edelstahl oder Titan hergestellt und damit sterilisierbar und wiederverwendbar.

Für die Markierung der Stellen an der Sklera, die für den operativen Zugang zur Retina ausgewählt wurden, kann entweder ein dafür geeigneter Stift mit einem geeigneten Farbstoff verwendet werden, oder geeignete Instrumente, die Marker oder Markeur genannt werden. Im Allgemeinen weisen diese Marker einen Handgriff auf und einen Fortsatz mit einem Ende, in das ein Farbstoff gefüllt ist oder das auf Grund seiner Geometrie einen Abdruck auf der Sklera hinterlässt. Ein Beispiel dafür ist der Sklera-Marker nach Gass. Marker enthalten entweder ein Reservoir des geeigneten Farbstoffes oder sie werden mit Druck auf die Sklera gepresst, wodurch ein für einige Zeit erkennbarer Abdruck auf der Sklera entsteht.

Für die Markierung mittels eines Farbstoffes werden verschiedene Farbstoffe verwendet, die pharmazeutisch akzeptabel und arzneimittelrechtlich zugelassen sein müssen. Beispielsweise können chirurgische Hautmarker-Farbstoffe verwendet werden. Diese Farbstoffe können auch bei der erfindungsgemäßen Vorrichtung eingesetzt werden.

Die bekannten Indentoren und Marker haben den Nachteil, dass zwei verschiedene Instrumente benötigt werden, was während des Gebrauchs bei dem operativen Eingriff am Auge und bei der anschließenden Reinigung einen erhöhten Zeitaufwand bedeutet.

Es gibt auch kombinierte augenchirurgische Instrumente, die als Indentoren und Marker verwendbar sind, beispielsweise der Depressor-Marker nach O'Connor. Dieses Instrument weist einen Handgriff mit zwei Fortsätzen auf. Ein Fortsatz hat ein Ende, welches für das Drücken auf die Sklera ausgebildet ist. Der andere Fortsatz ist wie bei dem Sklera-Marker nach Gass ausgebildet. Der Nachteil dieser Kombinationsinstrumente ist, dass sich die beiden Fortsätze an den gegenüberliegenden Enden des Handgriffes befinden. Deshalb muss das Instrument nach der Indentation aus der Hand gelegt und für die Markierung wieder in die Hand genommen werden. Dadurch leidet die Genauigkeit der Markierung, weil sich der operierende Arzt beim Wenden des Kombinationsinstrumentes die genaue Position der zu markierenden Stelle an der Sklera merken muss.

Alle Indentoren, also sowohl die Indentoren an sich als auch die Kombinationsinstrumente haben den Nachteil, dass trotz sorgfältiger Kontrolle der krankhaften Veränderungen an der Retina mittels indirekter Ophthalmoskopie die von ihnen verursachte Ausbuchtung an der Retina schlecht und nur ungenau zu erkennen ist. Das führt dazu, dass dann auch die Markierung nicht an der richtigen Stelle an der Sklera erfolgt.

Alle Marker, also sowohl die Farbstoffstifte, als auch die augenchirurgischen Instrumente haben folgende Nachteile: Die Markierung ist nicht immer gut erkennbar und abgrenzbar, weil der Farbstoff verläuft oder das Ende des Fortsatzes zu groß ist und damit ein zu großer Farbpunkt auf die Sklera appliziert wird. Außerdem lassen sich mit den bekannten Markern maximal einige wenige Punkte an der Sklera markieren, weil dann das Farbreservoir leer ist. Mit keinem der bekannten Markern lassen sich Linien auf der Sklera anbringen, was bei einigen Erkrankungen aber wünschenswert wäre, um die Ausdehnung des krankhaft veränderten Areals genau zu markieren.

Bei den Markern, die mechanisch einen Abdruck auf der Sklera verursachen, muss mit einem ausreichenden Druck auf die Sklera gedrückt werden, was zu Schäden an der Sklera führen kann. Dieser Abdruck ist aber auch bei starkem Druck nur für eine bestimmte kurze Zeit sichtbar, was bei zeitintensiven Eingriffen nachteilig ist. Der Abdruck ist also oftmals nur ungenau und für kurze Zeit zu erkennen.

### Aufgabe

Die Aufgabe der vorliegenden Erfindung ist es, ein augenchirurgisches Instrument zur Verfügung zu stellen, mit dem genau, schnell und lange haltbare Markierungen auf der Sklera anbringbar sind, durch die krankhafte Veränderungen an der Retina genau dargestellt werden können.

### Lösung der Aufgabe

Die erfindungsgemäße Aufgabe wird gelöst durch die Vorrichtung aus Anspruch 1.

Die erfindungsgemäße Vorrichtung 1 umfasst einen Handgriff 2, an dessen einen Ende sich ein Fortsatz 3 mit einer Spitze 4 befindet. Der Fortsatz 3 hat eine geeignete Länge, um eine Indentation und Markierung der Sklera durchzuführen. Der Fortsatz 3 kann gerade sein. In einer bevorzugten Ausführung ist der Fortsatz 3 gebogen, um besser die Zielstrukturen an der Sklera für die Indentation und Markierung zu erreichen. Die Biegung des Fortsatzes 3 beträgt vorzugsweise 10 bis 25 Winkelgrad.

Vorzugsweise ist der Handgriff 2 in einer ergonomisch vorteilhaften Weise ausgeführt, beispielsweise mit Einbuchtungen für die Finger oder einer geriffelten Oberfläche.

Die Spitze 4 ist so ausgebildet, dass damit sowohl die Indentation als auch die Markierung gleichzeitig durchgeführt werden können. Dazu weist die Spitze 4 ein Reservoir 8 für den Farbstoff auf, wobei das Reservoir 8 so groß ausgelegt ist, dass die Menge des Farbstoffes für eine Vielzahl von Markierungen (Punkte oder Linien) ausreicht. Das Reservoir 8 wird vor der Indentation und Markierung mit einem dafür geeigneten Farbstoff gefüllt.

An der Spitze 4 der Vorrichtung 1 endet auch die Markierungseinheit 10, welche durch einen Auslösemechanismus 7 in die Spitze 4 versenkbar ist (Position 1 bei der Indentation) oder aus der Spitze 4 herausfahrbar ist (Position 2 bei der Markierung). Befindet sich die Markierungseinheit 10 in der Position 1 (die Markierungseinheit ist in der Spitze 4 versenkt), dann wird keine Farbe von dem Reservoir 8 über die Markierungseinheit 10 auf die Sklera appliziert. In der Position 1 der Markierungseinheit dient die Vorrichtung 1 also nur der Indentation. Befindet sich die Markierungseinheit 10 in der Position 2 (die Markierungseinheit ragt über die Spitze 4 heraus), dann wird Farbe von dem Reservoir 8 über die Markierungseinheit 10 auf die Sklera appliziert. In der Position 2 dient die Vorrichtung 1 also sowohl der Indentation als auch der Markierung.

In einer bevorzugten Ausführungsform weist das Ende der Markierungseinheit 10 eine aufgeraute, geriffelte, mit Zacken versehene oder sonstige unregelmäßige Oberfläche auf, um den Farbstoff für die Markierung besser aufnehmen zu können.

Zwischen der Spitze 4 der Vorrichtung 1 und der Markierungseinheit 10 befindet sich ein kapillarer Spalt, der den Farbstoff für die Markierung aufnimmt. Dieser Spalt stellt also das Reservoir 8 dar. Es ist besonders vorteilhaft, wenn sowohl das Ende der Markierungseinheit 10 eine unregelmäßige Oberfläche aufweist als auch ein kapillarer Spalt sich zwischen der Spitze 4 der Vorrichtung 1 und der Markierungseinheit 10 befindet, weil damit eine größere Menge an Farbstoff für die Anfertigung vieler Markierungen während eines operativen Eingriffes zur Verfügung steht. Das ist besonders dann vorteilhaft, wenn nicht nur Punkte sondern auch Linien als Markierung auf der Sklera angebracht werden sollen.

Der Handgriff 2 umfasst einen Auslösemechanismus 7, mit dem über eine Positionsveränderung der Markierungseinheit 10 der Austritt des Farbstoffes an der Spitze 4 der Vorrichtung 1 ausgelöst werden kann. Der Auslösemechanismus 7 kann mechanisch oder elektronisch gesteuert und ausgelöst werden.

Über den Auslösemechanismus 7 kann beispielsweise die Markierungseinheit 10 in die Spitze 4 der Vorrichtung 1 versenkt werden (Position 1 der Markierungseinheit 10) oder die Markierungseinheit 10 ragt über die Spitze 4 der Vorrichtung 1 heraus (Position 2 der Markierungseinheit 10). Die Markierungseinheit 10 ist mit dem Auslösemechanismus 7 verbunden, so dass durch Betätigen des Auslösemechanismus 7 der Farbstoff aus dem Reservoir 8 gezielt und genau auf den zu markierenden Stellen appliziert werden kann. Wenn der Auslösemechanismus 7 während einer Bewegung der Vorrichtung 1 aktiviert ist, dann werden Linien auf der Sklera produziert. Wenn bei aktiviertem Auslösemechanismus 7 die Vorrichtung 1 nicht bewegt wird, dann wird ein Punkt auf der Sklera produziert.

In einer vorteilhaften Anordnung tritt aus dem Reservoir 8 an der Spitze 4 der Vorrichtung 1 der Farbstoff mittels Kapillarkräfte nach dem Prinzip einer Tinten-Füllfeder aus und kann damit auf die Sklera übertreten, so lange der Auslösemechanismus 7 aktiviert ist, das heißt, solange die Markierungseinheit 10 sich in der Position 2 befindet. Wenn der Auslösemechanismus 7 nicht aktiviert ist und sich somit die Markierungseinheit 10 in der Position 1 befindet, dann tritt kein Farbstoff aus dem Reservoir 8 an der Spitze 4 der Vorrichtung 1 aus und es wird keine Markierung an der Sklera erzeugt. Damit wird vermieden, dass unerwünschter Weise Farbstoff auf die Sklera gelangt, solange die richtige Position der Spitze 4 an der Sklera noch nicht erreicht ist. Die richtige Position wird durch die gleichzeitige Kontrolle der Retina mittels indirekter Ophthalmoskopie festgestellt.

Der Auslösemechanismus 7 kann über eine geeignete Mechanik die Markierungseinheit 10 zwischen den Positionen 1 und 2 bewegen. Alternativ ist es auch möglich, dass der Auslösemechanismus 7 über geeignete elektromechanische oder elektronische Bauteile die Markierungseinheit 10 zwischen den Positionen 1 und 2 bewegt. Unabhängig davon, auf welche Weise der Auslösemechanismus 7 die Markierungseinheit 10 bewegt, wird durch den Auslösemechanismus 7 das Reservoir 8 für den Farbstoff an der Spitze 4 der Vorrichtung 1 geöffnet, so dass Farbstoff aus der Spitze austreten kann und somit eine Markierung der Sklera ermöglicht wird.

Die Vorrichtung 1 umfasst vorzugsweise mindestens einen Kanal 5, der durch das Innere der Vorrichtung 1 geführt sein kann oder außen an der Vorrichtung 1 angeordnet ist. Alternativ kann der mindestens eine Kanal 5 auch teils durch das Innere der Vorrichtung 1 geführt sein und teils außen an der Vorrichtung 1 angeordnet sein. In allen Fällen ist der mindestens eine Kanal 5 so ausgeführt, dass durch ihn ein herkömmlicher Lichtleiter 11 an die Spitze 4 der Vorrichtung 1 geführt werden kann. Durch den mindestens einen Kanal 5 wird also über den Lichtleiter 11 Licht zur Spitze 4 der Vorrichtung 1 geleitet. Alternativ kann die Vorrichtung 1 auch mehrere Kanäle 5 umfassen, die jeweils einen Lichtleiter 11 an die Spitze 4 der Vorrichtung 1 führen. Ein bevorzugter Lichtleiter 11 ist eine Glasfaser.

Vorzugsweise weist der Kanal oder die Kanäle 5 einen Durchmesser von 23 bis 29 Gauge auf, weil herkömmliche Lichtleiter in der Augenchirurgie einen Durchmesser von ca. 0,4 mm aufweisen. Das eine Ende des Lichtleiters wird mit einem herkömmlichen vitreoretinalen Operationsset verbunden, welches eine Lichtquelle aufweist. Über den Lichtleiter 11 wird das Licht vom Operationsset durch den Kanal oder die Kanäle 5 bis an die Spitze 4 der Vorrichtung 1 geleitet, wo das Licht auf die Sklera abgestrahlt wird, und zwar genau an der Stelle, an der die Spitze 4 der Vorrichtung 1 die Sklera eindellt, also wo die Indentation stattfindet. Da dieses Licht von der Spitze 4 der Vorrichtung 1 durch die Sklera und die Chorioidea bis zur Retina dringt, kann die tatsächliche Stelle der Indentation mittels indirekter Ophthalmoskopie sehr genau festgestellt werden. Wenn die Indentationsstelle mit der krankhaft veränderten Stelle an der Retina genau übereinstimmt, dann wird diese Indentationsstelle an der Sklera markiert. Wenn die Indentationsstelle mit der krankhaft veränderten Stelle an der Retina nicht genau übereinstimmt, dann wird die Indentationsstelle so verändert, bis sie mit der krankhaft veränderten Stelle an der Retina genau übereinstimmt. Als Lichtquelle am Operationsset kann eine Xenonlichtquelle, eine LED oder eine andere herkömmliche Lichtquelle verwendet werden.

Die Zuführung von Licht durch einen oder mehrere Kanäle 5 an die Spitze 4 der Vorrichtung 1 ermöglicht also, dass die Markierungen an der Sklera genauer sind und verbessert daher die Erfolgsaussichten der vitreoretinalen Operation. Denn je genauer die Markierung auf der Sklera tatsächlich die krankhaft veränderte Stelle an der Retina oder der Chorioidea markiert, desto genauer kann der Arzt die krankhaft veränderten Stellen operativ behandeln.

Die Vorrichtung 1 umfasst vorzugsweise eine Verschlusseinheit 9, welche den in den Kanal 5 eingeführten Lichtleiter 11 an der Vorrichtung 1 fixiert, damit während des Einsatzes der Vorrichtung 1 der Lichtleiter 11 nicht verrutscht oder aus dem Kanal 5 herausrutscht. Analog dazu fixiert die Verschlusseinheit 9 eine Vielzahl von Lichtleitern 11, wenn eine Vielzahl von Kanälen 5 an der Vorrichtung 1 vorgesehen sind. Die Verschlusseinheit kann beispielsweise als Klemmverschluss oder Schraubverschluss ausgebildet sein oder in einer anderen geeigneten Form.

Die erfindungsgemäße augenchirurgische Vorrichtung umfasst eine Spitze 4, die sowohl für die Indentation der Sklera eingesetzt werden kann, als auch den Farbstoff für eine Vielzahl von Markierungen aufnehmen kann. Die erfindungsgemäße Vorrichtung 1 ist also ein kombinierter Sklera-Depressor-Marker.

In einer bevorzugten Ausführungsform weist jeder Kanal 5 für den Lichtleiter 11 an seinem Ende 6 an der Spitze 4 der Vorrichtung 1 eine Verengung auf, sodass der Lichtleiter 11 nicht an der Spitze 4 der Vorrichtung 1 austreten kann. Damit wird eine Verletzung der Sklera durch den Lichtleiter 11 vermieden. Diese Verengung kann konzentrisch oder asymmetrisch sein. Die Verengung kann kreisrund oder spaltförmig sein. Die Verengung kann zentral oder peripher am Ende 6 des Kanals 5 an der Spitze 4 der Vorrichtung 1 angeordnet sein. Es ist wesentlich, dass die Verengung so ausgebildet ist, dass einerseits der Lichtleiter 11 verlässlich am Durchtritt durch die Verengung gehindert wird aber andererseits eine ausreichende Lichtmenge durch die Verengung auf die Sklera trifft. Üblicherweise werden in der Augenchirurgie als Lichtleiter 11 Glasfasern verwendet. Da herkömmliche Glasfasern in der Augenchirurgie einen Durchmesser von ca. 0,4 mm aufweisen, muss die Verengung kleiner als 0,4 mm sein.

In einer bevorzugten Ausführungsform weist jeder Kanal 5 für einen Lichtleiter 11 an seinem der Spitze 4 abgewandtem Ende eine Verschlusseinheit 9 auf, mit der jeder Lichtleiter 11 an der Vorrichtung 1 fixiert werden kann, so dass sie nicht aus den Kanälen 5 herausrutschen können.

Durch die Beleuchtung der Spitze 4 der Vorrichtung 1 während der Indentation kann die genaue Position der Spitze 4 in Relation zur Retina jederzeit intraoperativ durch die indirekte Ophthalmoskopie kontrolliert werden. Sobald festgestellt wird, dass sich die Spitze 4 der Vorrichtung 1 an der richtigen Position befindet, wird diese Stelle mit dem Farbstoff, der im Reservoir 8 der Vorrichtung 1 gespeichert ist, markiert. Anschließend kann an der markierten Stelle der eigentliche operative Eingriff an der Retina durchgeführt werden.

Die erfindungsgemäße Vorrichtung 1 ist aus Edelstahl, Titan oder einem anderen geeigneten, sterilisierbaren und wiederverwendbaren Material hergestellt.

Der Fortsatz 3 mit der Spitze 4 soll einen Durchmesser aufweisen, der für die Indentation geeignet ist. Wenn der Durchmesser zu groß ist, dann entsteht bei der Indentation eine großflächige Auswölbung an der Retina, so dass kleine krankhafte Veränderungen an der Retina nicht mehr genau dargestellt werden können. Wenn der Durchmesser zu klein ist, dann entsteht bei der Indentation eine kleine Auswölbung an der Retina, die unter Umständen mittels der indirekten Ophthalmoskopie nicht mehr zu erkennen ist. Vorzugsweise beträgt der Durchmesser des Fortsatzes 3 und der Spitze 4 0,1 bis 4 mm. Alternativ kann die Spitze 4 einen größeren Durchmesser aufweisen als der Fortsatz 3.

Das Ende der Markierungseinheit 10 an der Spitze 4 soll einen Durchmesser aufweisen, der für die Markierung geeignet ist. Wenn der Durchmesser zu groß ist, dann entsteht bei der Markierung ein zu großer Punkt oder eine zu breite Linie. Dadurch wird die Genauigkeit der Markierung beeinträchtigt. Wenn der Durchmesser zu klein ist, dann entsteht bei der Markierung ein zu kleiner Punkt oder eine zu schmale Linie. Dadurch wird die Sichtbarkeit der Markierung beeinträchtigt. Vorzugsweise beträgt der Durchmesser des Endes der Markierungseinheit 10 an der Spitze 4 0,1 bis 4 mm. Jedenfalls ist der Durchmesser des Endes der Markierungseinheit 10 an der Spitze 4 etwas geringer als der Durchmesser des Fortsatzes 3 und der Spitze 4, damit ein Reservoir 8 für den Farbstoff zwischen der Markierungseinheit 10 und dem Fortsatz 3 und der Spitze 4 gebildet wird.

### Abbildungslegende

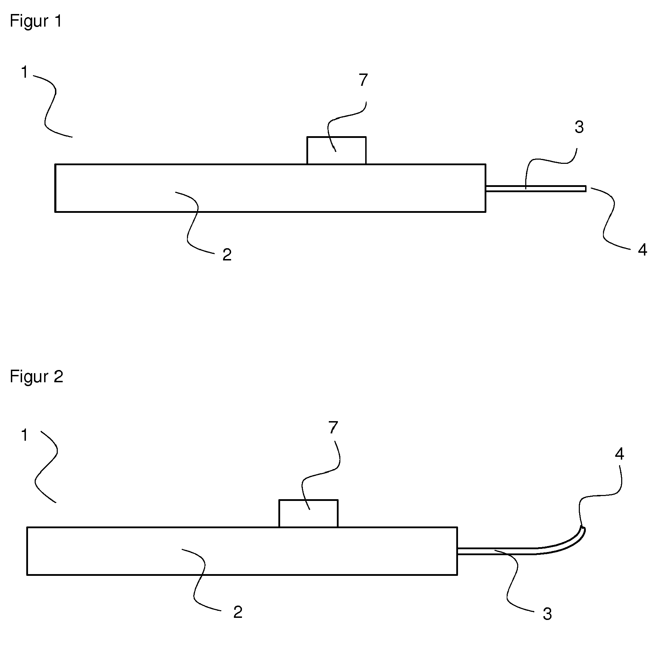

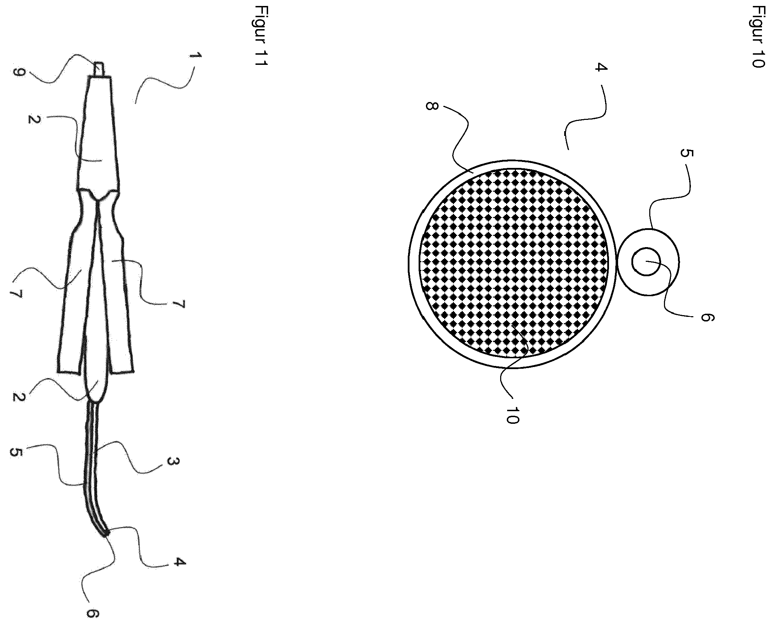

**Figur 1** zeigt eine Aufsicht auf die erfindungsgemäße Vorrichtung 1 mit dem Handgriff 1, einem geraden Fortsatz 3 mit Spitze 4 und einem Auslösemechanismus 7. Im Gegensatz zu den bekannten kombinierten Indentations- und Markierungsinstrumenten weist die erfindungsgemäße Vorrichtung 1 nur einen Fortsatz 3 auf, mit dem sowohl die Indentation als auch die Markierung durchgeführt werden kann. Dadurch ist die Handhabung für den Arzt während des operativen Eingriffes vereinfacht und beschleunigt.
   Die Markierungseinheit 10 ist in dieser Abbildung nicht sichtbar, weil sie sich innerhalb des Fortsatzes 3 in der Position 1 befindet, bei der die Markierungseinheit 10 in der Spitze 4 versenkt ist.
**Figur 2** zeigt ebenfalls eine Aufsicht auf die erfindungsgemäße Vorrichtung 1. Der Fortsatz 3 ist gebogen, wodurch eine leichtere und gezieltere Handhabung der Vorrichtung 1 für den Arzt erreicht wird.

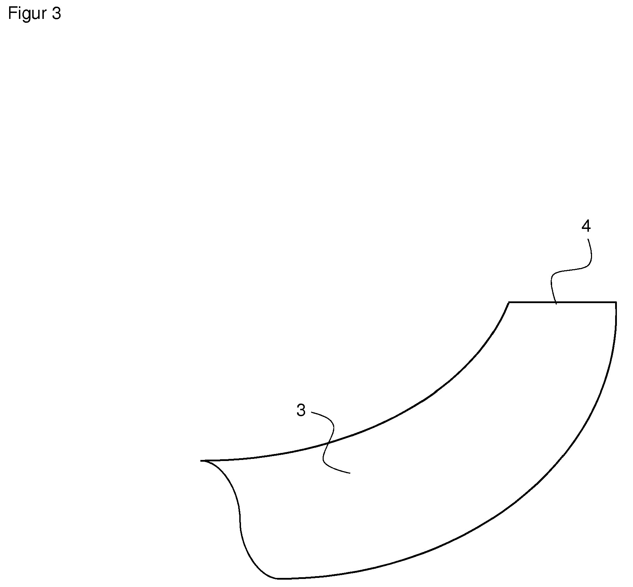

**Figur 3** zeigt eine vergrößerte Aufsicht auf den gebogenen Fortsatz 3 und die Spitze 4. Die Markierungseinheit 10 ist in dieser Abbildung nicht sichtbar, weil sie sich innerhalb des Fortsatzes 3 in der Position 1 befindet, bei der die Markierungseinheit 10 in der Spitze 4 versenkt ist.

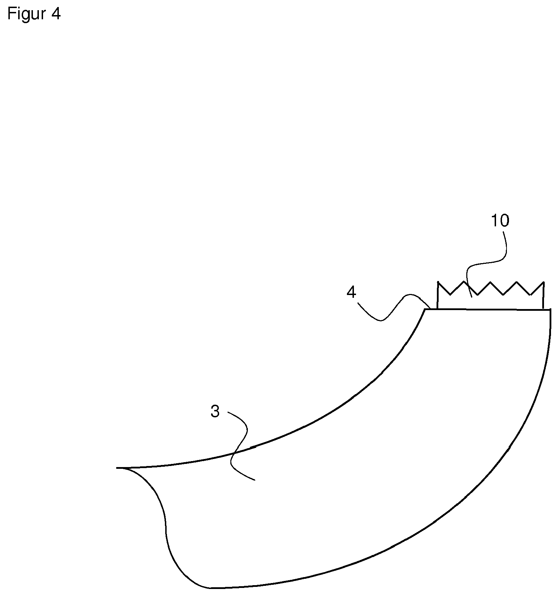

**Figur 4** zeigt ebenfalls eine vergrößerte Aufsicht auf den gebogenen Fortsatz 3 und die Spitze 4. Die Markierungseinheit 10 ist in dieser Abbildung sichtbar, weil sie sich in der Position 2 befindet, bei der die Markierungseinheit 10 über die Spitze 4 herausragt. Besonders vorteilhaft ist eine aufgeraute, geriffelte, mit Zacken versehene oder sonstige unregelmäßige Oberfläche am Ende der Markierungseinheit 10, um den Farbstoff für die Markierung besser aufnehmen und auf der Sklera verteilen zu können.

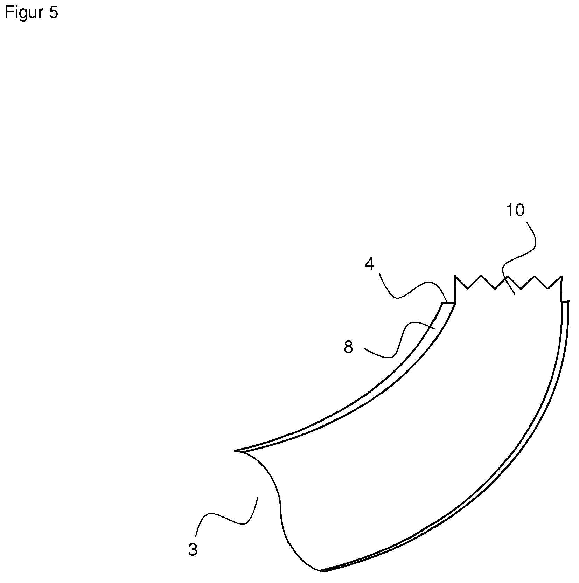

**Figur 5** zeigt einen Längsschnitt durch den gebogenen Fortsatz 3 mit der Spitze 4. Der Fortsatz 3 umhüllt die Markierungseinheit 10. Da der Durchmesser des Fortsatzes 3 mit der Spitze 4 geringfügig größer ist als der Durchmesser der Markierungseinheit 10 entsteht zwischen dem Fortsatz 3 und der Markierungseinheit 10 ein Reservoir 8 für Farbstoff.
   Die Markierungseinheit 10 ragt in dieser Abbildung über die Spitze 4 heraus, weil sie sich in der Position 2 befindet. In dieser Position fließt der Farbstoff auf Grund kapillarer Kräfte aus dem Reservoir 8 zur Spitze 4. Wenn die Vorrichtung 1 mit der Spitze 4 und dem Ende der Markierungseinheit 10 mit der unregelmäßigen Oberfläche auf die Sklera gedrückt wird, dann verursacht die Markierungseinheit in der Position 2 die Markierung der Sklera an dieser Stelle, weil der Farbstoff vom Reservoir 8 zur Spitze 4 fließt. Wenn die Markierungseinheit 10 sich in der Position 1 befindet, dann berührt sie nicht die Sklera, selbst wenn die Spitze 4 der Vorrichtung während der Indentation auf die Sklera gedrückt wird.

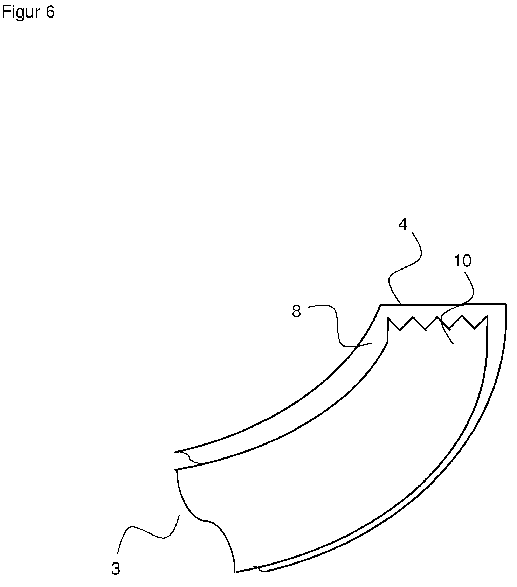

**Figur 6** zeigt einen Längsschnitt durch den gebogenen Fortsatz 3 mit der Spitze 4. Die Markierungseinheit 10 befindet sich in dieser Abbildung innerhalb des Fortsatzes 3 in der Position 1, bei der die Markierungseinheit 10 in der Spitze 4 versenkt ist. Das Reservoir 8 für den Farbstoff wird von dem Fortsatz 3 und der Markierungseinheit 10 begrenzt. Der Farbstoff wird vor Beginn des operativen Eingriffes in das Reservoir 8 gefüllt, wobei sich dafür die Markierungseinheit 10 in der Position 1 befindet.
   Wird die Spitze 4 bei versenkter Markierungseinheit 10 (also wenn die Markierungseinheit 10 sich in der Position 1 befindet) für die Indentation auf die Sklera gedrückt, dann gelangt kein Farbstoff auf die Sklera, weil der Abstand des Endes der Markierungseinheit 10 zur Spitze 4 zu groß ist, als dass durch Kapillarkräfte der Farbstoff bis zu Spitze 4 gelangen könnte. Erst wenn während der Indentation beim Erreichen der zu markierenden Stelle auf der Sklera mit dem Auslösemechanismus 7 (in dieser Abbildung nicht gezeigt) die Markierungseinheit 10 von der Position 1 in die Position 2 bewegt wird, dann gelangt durch die Markierungseinheit 10 Farbstoff auf die Sklera. Da die Spitze 4 und die Markierungseinheit 10 sich am bzw. im Fortsatz 3 befinden, ermöglicht die Vorrichtung 1 eine Indentation und Markierung ohne Instrumentenwechsel und damit in kürzerer Zeit.

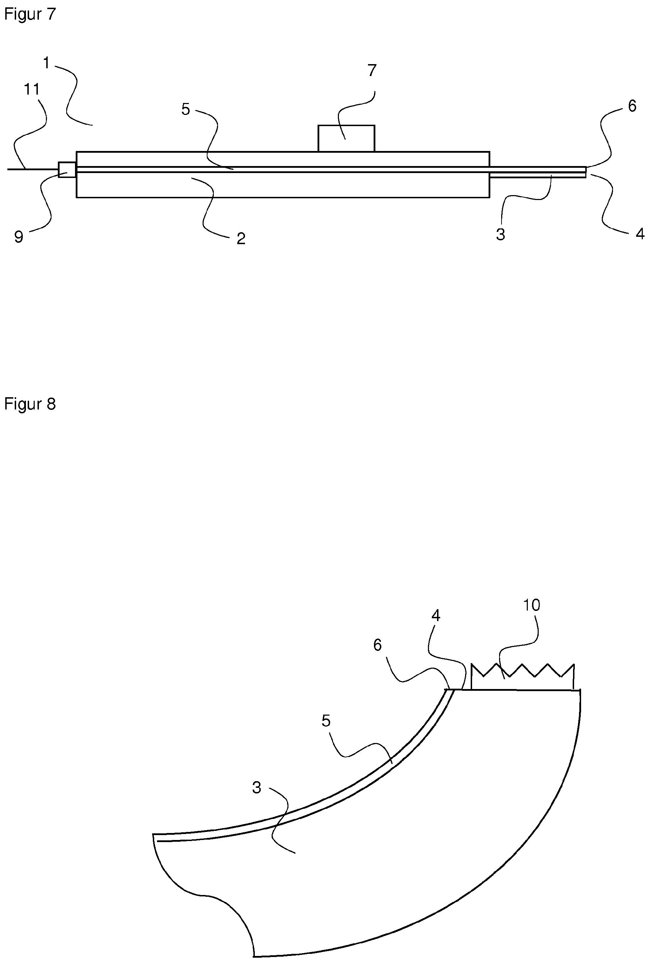

**Figur 7** zeigt eine Aufsicht auf die Vorrichtung 1. Die Markierungseinheit 10 ist in dieser Abbildung nicht sichtbar, weil sie sich innerhalb des Fortsatzes 3 in der Position 1 befindet, bei der die Markierungseinheit 10 in der Spitze 4 versenkt ist. Zusätzlich zu den in der Figur 1 dargestellten Merkmalen zeigt die Figur 7 einen Kanal 5, der außen am Handgriff 2 und am Fortsatz 3 angebracht ist. Das Ende 6 des Kanals 5 befindet sich an der Spitze 4 des Fortsatzes 3. Das andere Ende des Kanals befindet sich am abgewandten Ende des Handgriffs 2.
   Durch den Kanal 6 wird ein Lichtleiter 11 geführt, der durch das andere Ende des Kanals am abgewandten Ende des Handgriffs 2 eingeführt wird und bis zum Ende 6 des Kanals 5 vorgeschoben wird. Am Ende 6 weist der Kanal 5 eine Engstelle auf (in Figur 7 nicht dargestellt), damit der Lichtleiter 11 nicht durch das Ende 6 hindurchtreten kann. Der Durchmesser der Engstelle ist also kleiner als der Durchmesser des Lichtleiters 11. Die Engstelle ist aber ausreichend weit, damit das Licht, das vom Lichtleiter an das Ende 6 des Kanals 5 geführt wird, durch die Engstelle hindurchtreten kann und auf die Sklera leuchtet. Das Licht fällt durch die Sklera und die Chorioidea auf die Retina, wo es mittels indirekter Ophthalmoskopie leicht wahrgenommen werden kann. Diese Beleuchtung der Sklera während der Indentation ermöglicht eine wesentlich bessere Orientierung an der Retina während der indirekten Ophthalmoskopie. Dadurch kann die Position der Spitze 4 auf der Sklera eindeutig, schnell und einfach auf Grund der beleuchteten Ausbuchtung auf der Retina erkannt werden.
   Alternativ können auch mehrere Kanäle 5 an der Vorrichtung 1 angebracht werden, durch die dann jeweils ein oder mehrere Lichtleiter geführt werden können. Dadurch kann die Lichtstärke erhöht werden oder eine gleichmäßigere Ausleuchtung des beleuchteten Areals erzielt werden.
   Der mindestens eine Kanal 5 kann im Inneren durch die Vorrichtung 1 geführt werden, oder außen an der Vorrichtung 1 angebracht sein (wie in Figur 7 dargestellt), oder teils innen und teils außen (wie in Figur 11 dargestellt).
   Das Ende 6 des Kanals 5 muss sich an der Spitze 4 befinden, denn dort soll das Licht zur Beleuchtung der Sklera, der Chorioidea und der Retina hingeleitet werden. Das andere Ende des Kanals kann sich an einer beliebigen Stelle der Vorrichtung 1 befinden.
   Jenes Ende des Lichtleiters, das aus dem anderen Ende des Kanals 5 ragt, wird mit einer Lichtquelle (nicht gezeigt) verbunden, die das Licht zur Beleuchtung der Sklera, der Chorioidea und der Retina bereitstellt. Als Lichtquelle kann eine Xenonlichtquelle, eine LED oder eine andere herkömmliche Lichtquelle verwendet werden.
   Die Verschlusseinheit 9 bewirkt, dass der Lichtleiter 11 nach der vollständigen Einführung in den Kanal 5 in dieser Position fixiert wird, damit der Lichtleiter 11 während der Indentation und der Markierung nicht aus dem Kanal 5 herausrutscht. Die Verschlusseinheit kann eine Klammer, eine Schraube oder ein anderes geeignetes Bauteil sein.
**Figur 8** zeigt eine vergrößerte Aufsicht auf den gebogenen Fortsatz 3 und die Spitze 4. Die Markierungseinheit 10 ist in dieser Abbildung sichtbar, weil sie sich in der Position 2 befindet, bei der die Markierungseinheit 10 über die Spitze 4 herausragt. Besonders vorteilhaft ist eine aufgeraute, geriffelte, mit Zacken versehene oder sonstige unregelmäßige Oberfläche am Ende der Markierungseinheit 10, um den Farbstoff für die Markierung besser aufnehmen und auf der Sklera verteilen zu können.
   Zusätzlich zeigt die Figur 8 einen außen am Fortsatz 3 angebrachten Kanal 5 und dessen Ende 6 an der Spitze 4. Die Verengung am Ende 6 des Kanals 5 ist in dieser Darstellung nicht zu sehen. Der Lichtleiter 11 im Kanal 5 ist in dieser Darstellung nicht zu sehen.

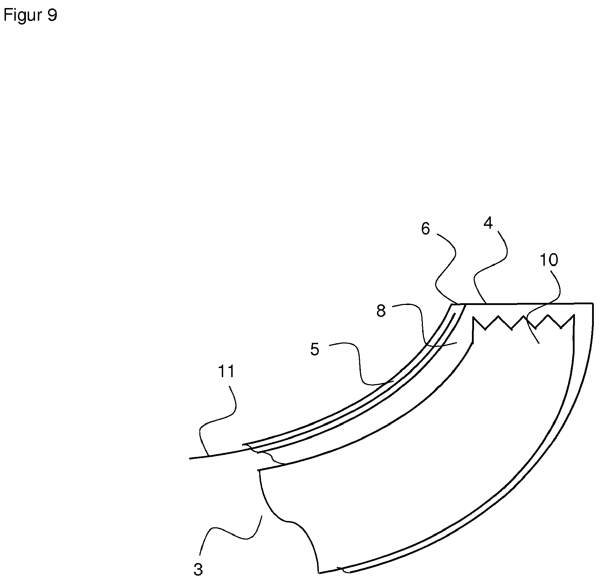

**Figur 9** zeigt einen Längsschnitt durch den gebogenen Fortsatz 3 mit der Spitze 4 und durch den Kanal 5 mit seinem Ende 6 an der Spitze 4. Im Kanal 5 ist ein Lichtleiter 11 bis kurz vor dem Ende 6 eingeführt. Die Verengung am Ende 6 des Kanals 5 ist in dieser Darstellung nicht zu sehen.
**Figur 10** zeigt eine Aufsicht auf die Spitze 4 der Vorrichtung 1 und auf das Ende 6 des Kanals 5. Das Reservoir 8 für den Farbstoff befindet sich zwischen der äußeren Begrenzung der Spitze 4 und der Markierungseinheit 10. Das in dieser Aufsicht erkennbare Ende der Markierungseinheit 10 hat eine aufgeraute, geriffelte, mit Zacken versehene oder sonstige unregelmäßige Oberfläche, damit der Farbstoff sich besser über die gesamte Oberfläche verteilt und bei der Markierung homogene und scharf begrenzte Punkte oder Linien auf der Sklera macht. Wenn während der Markierung die Vorrichtung über die Sklera geführt wird, dann ermöglicht die aufgeraute, geriffelte, mit Zacken versehene oder sonstige unregelmäßige Oberfläche der Markierungseinheit 10 das Anfertigen von Linien auf der Sklera. Dadurch können größere krankhafte Veränderungen umrandet werden. Eine solche linienförmige Markierung ist von Vorteil bei dem anschließenden operativen Eingriff, weil das Ausmaß und die Größe der krankhaften Veränderungen besser zu erkennen sind.
   In dieser Aufsicht ist am Ende 6 des Kanals 5 eine zentrale, runde Einengung zu erkennen, welche verhindert, dass der Lichtleiter 11 durch das Ende 6 hervortritt und eine Schädigung der Sklera während der Indentation und Markierung verursacht. Diese Einengung kann zentral oder peripher liegen, sie kann rund oder spaltförmig sein oder in einer sonstigen Ausführung vorliegen. Es ist nur wesentlich, dass die Einengung eng genug ist, um den Durchtritt des Lichtleiters 11 (in der Figur nicht dargestellt) zu verhindern, aber trotzdem weit genug ist, um eine ausreichende Menge Licht für die Beleuchtung der Sklera, der Chorioidea und der Retina während der Indentation und Markierung durchzulassen.
**Figur 11** zeigt ein anderes Ausführungsbeispiel der erfindungsgemäßen Vorrichtung. Der Kanal 5 ist im Bereich des Handgriffs 2 innerhalb der Vorrichtung geführt, im Bereich des Fortsatzes 3 außen an der Vorrichtung angebracht.

Der Auslösemechanismus 7 ist als Drückmechanismus mit zwei Flügeln ausgebildet. Durch Drücken auf die beiden Flügel wird die Markierungseinheit 10 in die Position 2 bewegt. Bei Entlastung der beiden Flügel wird durch einen Federmechanismus (nicht in der Figur gezeigt) die Markierungseinheit 10 wieder in die Position 1 zurück bewegt. Da die beiden Flügel mit der Hand umfasst werden, ermöglicht diese Anordnung eine gut und einfach dosierbare Abgabe des Farbstoffes aus dem Reservoir 8 (nicht in der Figur gezeigt).

### Bezugszeichenliste

- 1: Vorrichtung zur Indentation und Markierung der Sklera
- 2: Handgriff
- 3: Fortsatz
- 4: Spitze
- 5: Kanal
- 6: Ende des Kanals an der Spitze
- 7: Auslösemechanismus
- 8: Reservoir für Farbstoff
- 9: Verschlusseinheit
- 10: Markierungseinheit
- 11: Lichtleiter

## Patentansprüche

1. Vorrichtung (1) zur Indentation und Markierung der Sklera für die Behandlung von Erkrankungen der Retina eines Auges, umfassend
- einen Handgriff (2), geeignet zum Halten der Vorrichtung (1) mit einer Hand,
- einen Fortsatz (3) mit einer Spitze (4), geeignet für die Durchführung einer Indentation,
- eine Markierungseinheit (10) mit einem Auslösemechanismus (7), geeignet für die Durchführung einer Markierung
wobei
- der Fortsatz (3) mit der Spitze (4) an einem Ende des Handgriffs (2) angebracht ist,
- die Spitze (4) ein Reservoir (8) für einen Farbstoff aufweist,
- die Markierungseinheit (10) an der Spitze (4) endet,
- die Markierungseinheit (10) sich in einer Position 1 befindet, in der Farbstoff aus dem Reservoir (8) an der Spitze (4) der Vorrichtung (1) nicht austreten kann,
- der Auslösemechanismus (7) an einer geeigneten Stelle am Handgriff (2) angebracht ist,
**dadurch gekennzeichnet, dass** bei der Aktivierung des Auslösemechanismus (7) die Markierungseinheit (10) in eine Position 2 bewegt wird, wodurch der Farbstoff aus dem Reservoir (8) an der Spitze (4) der Vorrichtung (1) austritt.

2. Vorrichtung nach Anspruch 1, **dadurch gekennzeichnet, dass** das Ende der Markierungseinheit (10) an der Spitze (4) eine aufgeraute, geriffelte, mit Zacken versehene oder sonstige unregelmäßige Oberfläche aufweist.

3. Vorrichtung nach Anspruch 1, **dadurch gekennzeichnet, dass** das Reservoir (8) an der Spitze (4) als kapillarer Spalt zwischen der Spitze (4) und der Markierungseinheit (10) ausgebildet ist.

4. Vorrichtung nach einem der vorhergehenden Ansprüche, **dadurch gekennzeichnet, dass** die Vorrichtung (1) mindestens einen Kanal (8) aufweist und so ausgeführt ist, dass durch den mindestens einen Kanal (8) mindestens ein Lichtleiter (11) an die Spitze (4) der Vorrichtung (1) geführt werden kann.

5. Vorrichtung nach Anspruch 4, **dadurch gekennzeichnet, dass** das Ende (6) des mindestens einen Kanals (5) an der Spitze (4) der Vorrichtung (1) eine Verengung aufweist.

6. Vorrichtung nach Anspruch 5, **dadurch gekennzeichnet, dass** die Verengung konzentrisch oder asymmetrisch ist, rund oder spaltförmig ist und zentral oder peripher angeordnet ist.

7. Vorrichtung nach einem der vorhergehenden Ansprüche, **dadurch gekennzeichnet, dass** die Vorrichtung (1) mindestens eine Verschlusseinheit (9) aufweist, die so ausgebildet ist, dass der in den Kanal (5) eingeführte Lichtleiter (11) an der Vorrichtung (1) fixiert werden kann.

8. Vorrichtung nach Anspruch 7, **dadurch gekennzeichnet, dass** die Verschlusseinheit (9) als Klemmverschluss oder Schraubverschluss ausgebildet ist.
